# EUROPEAN PATENT SPECIFICATION

(11) **EP 4 316 460 B1**
(45) Date of publication and mention of the grant of the patent: **03.09.2025**
(21) Application number: 23189449.4
(22) Date of filing: 03.08.2023
(51) Int. Cl.: A61K 8/64, A61K 8/67, A61K 8/9789, A61Q 19/08

(54) **COSMETIC COMPOSITIONS COMPRISING A SYNERGIC MIX OF ACTIVE INGREDIENTS HAVING ANTIAGING ACTIVITY**
KOSMETISCHE ZUSAMMENSETZUNGEN, DIE EINE SYNERGISCHE MISCHUNG VON WIRKSTOFFEN MIT ALTERUNGSSCHUTZWIRKUNG ENTHALTEN
COMPOSITIONS COSMÉTIQUES COMPRENANT UN MÉLANGE SYNERGIQUE D'INGRÉDIENTS ACTIFS AYANT UNE ACTIVITÉ ANTI-ÂGE

(30) Priority: 04.08.2022 IT 202200016632
(43) Date of publication of application: 07.02.2024
(73) Proprietor: Unifarco S.p.A., 32035 Santa Giustina (BL) (IT)
(72) Inventor: Baratto, Giovanni, 32035 Santa Giustina BL (IT); Busata, Laura, 32035 Santa Giustina BL (IT); Moretti, Marta, 32035 Santa Giustina BL (IT)
(74) Representative: Perani & Partners S.p.A.

(56) References cited:
- EP-A1- 1 625 150
- DE-A1- 102018 105 686
- DATABASE GNPD [online] MINTEL; 28 June 2021 (2021-06-28), ANONYMOUS: "Skin Paste", XP093027541, retrieved from https://www.gnpd.com/sinatra/recordpage/8739805/ Database accession no. 8739805
- DATABASE GNPD [online] MINTEL; 31 July 2014 (2014-07-31), ANONYMOUS: "Self Tanner Body Butter", XP093027554, retrieved from https://www.gnpd.com/sinatra/recordpage/2543299/ Database accession no. 2543299

## Description

### FIELD OF THE INVENTION

The present invention relates to cosmetic compositions comprising active ingredients for the prevention and treatment of skin ageing.

### BACKGROUND OF THE INVENTION

Skin ageing, characterized by cumulative changes in its structure, function and tissue appearance, is a complex process influenced by intrinsic and extrinsic factors.

Intrinsic or chronological skin ageing typically occurs in areas not exposed to sunlight, revealing the influence of genetic factors. Photo-ageing, also called extrinsic ageing, is mainly caused by ultraviolet (UV) radiation and occurs mainly on the face and hands due to frequent exposure to sunlight and other factors such as air pollution and cigarette smoke.

In general, alterations in skin structure, function, and appearance are more evident in photo-aged skin with respect to chronologically-aged skin. However, these two types of ageing are difficult to separate and often find overlaps, since they have clinical characteristics in common caused by dermal matrix alterations which contribute to the formation of wrinkles, loss of elasticity and skin fragility [Structural and Functional Changes and Possible Molecular Mechanisms in Aged Skin. Lee, Hyunji, Yongiun Hong, and Miri Kim. 22, s.l. : International Journal of Molecular Sciences, 2021, Vol. 22*].*

Regardless of the type of ageing, wrinkles and reduced elasticity are typical phenomena of skin ageing and are the result of the progressive atrophy of the dermis. One of the main mechanisms of dermal atrophy is the reduction of the amount of extracellular matrix (ECM), in particular collagen. In aged skin, collagen production decreases and its degradation increases [*Molecular Mechanisms of Dermal Aging and antiaging approaches.* Shin, Jung-Won, et al. 20, s.l. : International journal of molecular sciences, 2019, Vol. 9*].*

Collagen is one of the most abundant proteins in many living organisms because it carries out a connective role in biological structures. It is also the most abundant protein in the extracellular matrix (ECM). There are four structural levels of the collagen protein: primary structure (amino acid triplet), secondary structure (helix), tertiary structure (triple helix), and quaternary structure (fibrils). Collagen is formed by a triple helix, the chains of which form a tight and stable structure. Different types of collagen are classified based on the composition of the α-chain. To date, 28 types of collagen have been identified and the most abundant is type I collagen, which represents more than 90% of the collagen present in the human body. Type I collagen is also the main collagen at the skin level (85-90% of skin collagen). The remaining is type III collagen (10-15%) [A Review of the Effects of Collagen Treatment in Clinical Studies. Wang, Hsiuying. 13, s.l. : Polymers, 2021, Vol. 22*].*

Collagen is produced by fibroblasts, cells present in the dermis which are also responsible for the production of elastin and glycosaminoglycans (GAGs). Fibroblasts are sensitive to mechanical stresses of the extracellular matrix (ECM) in which they are immersed and to biochemical and signaling stimuli, which can induce the activation and proliferation of such cells. Collagen fibres form a robust network which gives the dermis tonicity and elasticity.

Collagen and elastin, two of the main constituents of the extracellular matrix (ECM), change during the life cycle. Collagen production begins in the uterus, around the fifth week of the first trimester of pregnancy, at which time thin collagen fibres can be seen in the developing foetus. Subsequently, the collagen matrix increases and is associated with larger fibrils which begin to assemble into larger bundles. During childhood, through the stages of pre-puberty growth and puberty changes related to the production of sex hormones in adolescents, rapid and extensive collagen turnover occurs. Most publications on collagen production in pre-puberty and puberty adolescents are concerned with type I collagen, used as a supporting organic matrix which is mineralized for bone development. Collagen is constantly synthesized, deposited in the ECM, to then be degraded by enzymes, particularly MMPs (Matrix Metal Proteinase), in a balanced process which allows growth. Collagen synthesis and degradation are finely controlled and bio-chemically complex processes: they are fundamental for the development of tissues, their repair following damage and their maintenance in various anatomical and physiological systems [Skin collagen through the lifestages: Importance for skin health and beauty. Reilly, David M., and Jennifer Lozano. 2, s.l. : Plastic and Aesthetic Research, 2021, Vol. 8*].*

In the dermis of aged skin, a decrease in the density, thickness and degree of organization of dermal collagen is observed. Over the years, therefore, quantitative and structural changes of collagen fibres are found.

Young skin has collagen fibres which are intact, abundant and well organized, while in aged skin they are fragmented and unevenly distributed.

From the literature, a sharp decrease in collagen synthesis is noted after menopause, and it has been observed that dermal collagen production decreases with ageing, and the amount of collagen in 80-year-olds is 75% lower than in young adults. [Collagen fragmentation promotes oxidative stress and elevates matrix metalloproteinase-1 in fibroblasts in aged human skin. Fisher, Gary J, et al. 174: The American journal of pathology, 2009, Vol. 1]

Furthermore, from early adulthood, fibroblasts become less productive and collagen production decreases. From the literature, a single 1975 study estimated that collagen production decreases on average 1-1.5% per year.

A 'vicious cycle' has been hypothesized: the degradation of collagen leads to the loss of skin rigidity and resilience, which are clinically manifested as wrinkles. High molecular weight collagen fragments inhibit the synthesis of new collagen. Furthermore, collagen fragmentation by MMPs increases oxidative levels within damaged cells, which contributes to skin ageing and fibroblast damage.

There are numerous raw materials on the market capable of acting on the ageing mechanisms just described.

*Kigelia africana* is a tree native to Africa. The plant stem can reach 15 metres in height. From its fruits, which can weigh up to 10 kg, an extract is obtained for cosmetic use which is rich in peptides.

*Quillaja saponaria* has characteristic white flowers. Its dried bark produces a powder which is very rich in flavonoids and sugars such as glucose, galactose and rhamnose.

To pomegranate flower extract has been attributed a powerful action at the cutaneous level. In fact, the active ingredient is an effective antioxidant, promotes collagen synthesis and prevents skin damage due to exposure to UVA rays.

Furthermore, the extract is effective in inhibiting collagenase and elastase enzymes, showing an articulated anti-ageing action.

Vitamin C derivatives show a strong antioxidant activity, stimulate the production of collagen and promote the colour uniformity, reducing skin discolourations.

EP 1 625 150 B9 describes tripeptides for cosmetic use which can be useful in counteracting both intrinsic and extrinsic skin ageing.

The structure of these small tripeptides mimics the portion of the thrombospondin-1 (TSP-1) protein which binds to the complex formed between LAP (latency associated peptide) and TGF-β (Transforming Growth Factor-beta). This bond between TPS-1 and the complex releases the active version of TGF-β.

TGF-β is a key molecule in collagen homoeostasis. In fact, it:
- Promotes collagen production
- Prevents its degradation, inhibiting the production of matrix metal proteinase.

Physiological skin ageing and some exogenous factors such as exposure to solar radiation stimulate the production of free radicals. These inhibit the production of TGF-β and consequently collagen. At the same time, enzymes responsible for collagen metabolism such as MMPs are activated. All this accelerates the wrinkle formation process.

### SUMMARY OF THE INVENTION

The applicant has now selected cosmetic-grade ingredients which, appropriately combined, contribute to the production of cosmetic compositions which are surprisingly effective in reducing the visibility of wrinkles and improving the appearance of facial skin, thanks to the synergistic action of these active ingredients which act with a broad spectrum of action, i.e. capable of acting on the production and maintenance of collagen and proteins related to skin ageing.

The object of the present invention is therefore cosmetic compositions comprising:
a) sodium ascorbyl phosphate,
b) a pomegranate flower extract
c) a powdered extract of *Quillaja saponaria* bark
d) an extract from the fruit of *Kigelia africana*
e) at least one tripeptide amide of formula Lys-Val-Lys with one or more C12-C18 straight-chain fatty acids.

### DETAILED DESCRIPTION OF THE INVENTION

For the purposes of the present invention, the expression "consisting of" does not exclude the presence of further components beyond those expressly mentioned after such an expression.

The expression "consisting of" instead excludes the presence of further components, beyond those expressly listed after such an expression.

In the cosmetic compositions object of the present invention preferably the sum of the quantities of a) sodium ascorbyl phosphate, b) a pomegranate flower extract, c) a powdered extract of *Quillaja saponaria* bark, d) an extract from the fruit of *Kigelia africana,* e) at least one tripeptide amide of formula Lys-Val-Lys with one or more C12-C18 straight-chain fatty acids is between 0.02 and 0.5% by weight on the total weight of said cosmetic compositions.

For the purposes of the present invention, an example of a pomegranate extract suitable for making the cosmetic composition is the cosmetic ingredient sold under the name Colla-Gain^{®} by the company Lipoid Kosmetik AG.

For the purposes of the present invention, an example of powdered *Quillaja Saponaria* bark and an extract from the fruit of *Kigelia africana* suitable for making the cosmetic composition are those associated in the cosmetic ingredient sold under the name Dermotenseur^{™} by the company Gattefossé.

The cosmetic compositions object of the present invention are preferably in the form of cream or serum.

The term 'cream' is intended as an oil-in-water (O/W) or water-in-oil (W/O) emulsion having the features known to any cosmetic formulator.

The term 'serum' is intended as an O/W or W/O emulsion characterized by a higher concentration of active ingredients with respect to the 'cream' formulation.

According to a preferred embodiment, the cosmetic compositions in accordance with the invention are in the form of a cream and the sum of the quantities of a) sodium ascorbyl phosphate, b) a pomegranate flower extract, c) a powdered extract of *Quillaja saponaria* bark, d) an extract from the fruit of *Kigelia africana,* e) at least one tripeptide amide of formula Lys-Val-Lys with one or more C12-C18 straight-chain fatty acids is between 0.06 and 0.08% by weight, preferably it is equal to 0.0701% by weight on the total weight of said compositions.

According to another preferred embodiment, the cosmetic compositions in accordance with the invention are in the form of a serum and the sum of the quantities of a) sodium ascorbyl phosphate, b) a pomegranate flower extract, c) a powdered extract of *Quillaja saponaria* bark, d) an extract from the fruit of *Kigelia africana,* e) at least one tripeptide amide of formula Lys-Val-Lys with one or more C12-C18 straight-chain fatty acids is between 0.09% 0.2%, preferably it is equal to 0.15% by weight on the total weight of said compositions.

Preferably, the at least one tripeptide amide of formula Lys-Val-Lys of one or more C12-C18 straight-chain fatty acids is an amide of the above tripeptide with a C12-C18 straight-chain fatty acid. Still preferably, it is the tripeptide amide Lys-Val-Lys with palmitic acid.

According to a preferred embodiment, the tripeptide amide Lys-Val-Lys with palmitic acid is N-Palmitoyl-L-lysyl-L-valyl-L-lysine trifluoroacetate (1:2), having the formula I:

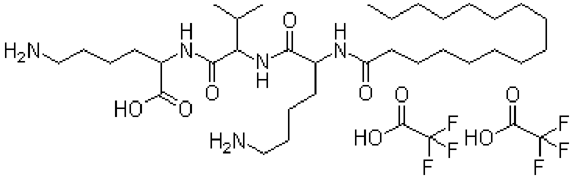

### Formula I

Preferably, the cosmetic compositions object of the invention further comprise (f) hyaluronic acid or a biologically acceptable salt thereof.

Preferably, the biologically acceptable salt of hyaluronic acid is an alkali metal salt, and further preferably is a sodium salt.

The hyaluronic acid is selected from the following types for the purposes of the present invention:
- hyaluronic acid with a weight average molecular weight of < 10,000 Da,
- hyaluronic acid with a weight average molecular weight between 10,000 and 1,000,000 Da,
- hyaluronic acid with a weight average molecular weight > 1,000,000 Da,
or mixtures thereof, of at least two of the above types.

The cosmetic compositions object of the present invention can comprise one, two or all three types of hyaluronic acid, and the total concentration of hyaluronic acid (regardless of the type or of the mixtures obtainable between the aforementioned types) is preferably between 0.01% and 1% by weight, on the total weight of the cosmetic compositions.

Thereby, an action on the entire skin barrier is guaranteed.

In fact, by employing:
- a high molecular weight hyaluronic acid (> 1,000,000 Da), a lifting and moisturising action is obtained on the dermal surface,
- a medium molecular weight hyaluronic acid (10,000 < x < 1,000,000 Da), a moisturising and anti-wrinkle action is obtained at the dermis-epidermal junction;
a low molecular weight hyaluronic acid (< 10,000 Da), a deeper moisturising action and stimulation of skin cell metabolism is obtained.

In the cosmetic compositions according to the present invention, the weight ratio between (a) sodium ascorbyl phosphate and (b) a pomegranate flower extract is preferably equal to 1:1.

In the cosmetic compositions according to the present invention, the weight ratio between the components (c) a powdered extract of *Quillaja saponaria* bark and (d) an extract from fruits of *Kigelia africana* is preferably equal to 1:1.

According to a preferred embodiment, the cosmetic composition according to the invention further comprises an active ingredient (g), to be added to the components a) sodium ascorbyl phosphate, b) a pomegranate flower extract, c) a powdered extract of Quillaja saponaria bark, d) an extract from fruits of Kigelia africana, e) at least one tripeptide amide of formula Lys-Val-Lys with one or more C12-C18 straight-chain fatty acids, and optionally f) hyaluronic acid or a biologically acceptable salt thereof.

Preferably, the active ingredient (g) is selected from the group consisting of: ascorbyl glucoside, N-acetyl-L-lysyl-L-alpha-aspartyl-L-valyl-L-tyrosine, *Akebia quinata* extract, niacinamide and *Salvia miltiorrhiza* leaf extract.

Each active ingredient of those listed above can be added to the cosmetic compositions object of the present invention, in the following percentages by weight on the total weight:
- ascorbyl glucoside: 0.5%;
- N-acetyl-L-lysyl-L-alpha-aspartyl-L-valyl-L-tyrosine: 0.001%;
- *Akebia quinata* extract: 0.025%;
- *Salvia miltiorrhiza* leaf extract: 0.008% and
- Niacinamide: 0.036%.

The cosmetic compositions object of the present invention are intended for non-therapeutic use in the prevention of skin ageing.

In particular, when the cosmetic compositions according to the present invention comprise as an active ingredient (g) ascorbyl glucoside, they are preferably used as an antioxidant to firm the skin.

According to another preferred solution, when the cosmetic compositions comprise as active ingredient (g) N-acetyl-L-lysyl-L-alpha-aspartyl-L-valyl-L-tyrosine, they are preferably used to firm the face and neck.

According to another preferred solution, when they contain as active ingredient (g) niacinamide and *Salvia miltiorrhiza* leaf extract, they are preferably used to firm and nourish the skin.

According to another preferred embodiment, when the cosmetic compositions contain as active ingredient (g) *Akebia quinata* extract, they are used as lifting ingredients to firm the skin.

Some cosmetic compositions object of the present invention are given for illustrative but non-limiting purposes.

### EXAMPLE 1: FACIAL SERUM

| **Component** | **range [% w/w]** |
|---|---|
| Aqua | as needed up to 100 |
| Wetting agents (Glycerin, propylene glycol, butylene glycol) | 1-10 |
| Oils (vegetable or synthetic), Waxes, Butters (Theobroma Cacao Seed Butter, Butyrospermum parkii butter, alba wax, Caprylic/Capric triglyceride) | 1-20 |
| Rheology modifiers (Polyacrylate crosspolymer-6, Carbomer, xanthan gum) | 1-5 |
| Emulsifiers (C14-22 Alcohols, C12-20 Alkyl Glucoside, PEG-100 stearate, potassium cethyl phosphate) | 0.5-4 |
| Propanediol | 0.00512-7.68 |
| Maltodextrins | 0.25-5 |
| Glycerine | 0.59-2.95 |
| Preservatives (Potassium sorbate, sodium benzoate, phenoxyethanol) | 0.1-1.5 |
| Stabilizers (chelators, antioxidants) (disodium EDTA, Pentaerythrityl tetra-di-t-butyl hydroxyhydrocinnamate, tocopherol) | 0.1-2 |
| Perfume | 0.1-1 |
| a) sodium ascorbyl phosphate | **0.008-0.16%** |
| b) a pomegranate flower extract | **0.008-0.16%** |
| c) a powdered extract of *Quillaja saponaria* bark | **0.00004-0.06%** |
| d) an extract from the fruit of *Kigelia africana* | **0.00004-0.06%** |
| e) at least one tripeptide amide of formula Lys-Val-Lys with one or more C12-C18 straight-chain fatty acids | **0.008-0.04%** |
| f) sodium hyaluronate | **0.01-1%** |

### EXAMPLE 2: O/W CREAM:

| **Component** | **range [% w/w]** |
|---|---|
| Aqua | as needed up to 100 |
| Oils (vegetable or synthetic), Waxes, Butters (Theobroma Cacao Seed Butter, Butyrospermum parkii butter, alba wax, Caprylic/Capric triglyceride) | 5-40 |
| Silicones (Dimethicone, Dimethicone crosspolymer, dimethiconol) | 1-10 |
| Wetting agents (Glycerin, propylene glycol, butylene glycol) | 1-10 |
| Rheology modifiers (Polyacrylate crosspolymer-6, Carbomer, xanthan gum) | 1-5 |
| Emulsifiers (C14-22 Alcohols, C12-20 Alkyl Glucoside, PEG-100 stearate, potassium cethyl phosphate) | 0.5-5 |
| Propanediol | 0.00512-7.68 |
| Maltodextrins | 0.25-5 |
| Glycerine | 0.59-2.95 |
| Preservatives (Potassium sorbate, sodium benzoate, phenoxyethanol) | 0.1-1.5 |
| Stabilizers (chelators, antioxidants) (disodium EDTA, Pentaerythrityl tetra-di-t-butyl hydroxyhydrocinnamate, tocopherol) | 0.1-2 |
| Perfume | 0.1-1 |
| a) sodium ascorbyl phosphate | **0.008-0.16%** |
| b) a pomegranate flower extract | **0.008-0.16%** |
| c) a powdered extract of *Quillaja saponaria* bark | **0.00004-0.06%** |
| d) an extract from the fruit of *Kigelia africana* | **0.00004-0.06%** |
| e) at least one tripeptide amide of formula Lys-Val-Lys with one or more C12-C18 straight-chain fatty acids | **0.008-0.04%** |
| f) sodium hyaluronate | **0.01-1%** |
| g) active ingredient¹ | **0.001-0.5%** |

### EXAMPLE 3: W/O EMULSION

| **Component** | **range [% w/w]** |
|---|---|
| Aqua | as needed up to 100 |
| Oils (vegetable or mineral) Waxes, fats (e.g., long chain alcohols) (Theobroma Cacao Seed Butter, Butyrospermum parkii butter, alba wax, Caprylic/Capric triglyceride) | 10-60 |
| Wetting agents (Glycerin, propylene glycol, butylene glycol) | 1-10 |
| Lipo rheology modifiers (Hydrogenated microcrystalline wax, hectorite, Synthetic wax) | 1-5 |
| Emulsifiers (PEG-30 dipolyhydroxystearate, Polyglyceryl-6 polyricinoleate, Polyglyceryl-6 polyhydroxystearate) | 0.5-5 |
| Propanediol | 0.00512-7.68 |
| Maltodextrins | 0.25-5 |
| Glycerine | 0.59-2.95 |
| Preservatives (Potassium sorbate, sodium benzoate, phenoxyethanol) | 0.1-1.5 |
| Perfume | 0.1-1 |
| Stabilizers (Magnesium sulfate, Magnesium stearate) | 0.1-2 |
| a) sodium ascorbyl phosphate | **0.008-0.16%** |
| b) a pomegranate flower extract | **0.008-0.16%** |
| c) a powdered extract of *Quillaja saponaria* bark | **0.00004-0.06%** |
| d) an extract from the fruit of *Kigelia africana* | **0.00004-0.06%** |
| e) at least one tripeptide amide of formula Lys-Val-Lys with one or more C12-C18 straight-chain fatty acids | **0.008-0.04%** |
| f) sodium hyaluronate | **0.01-1%** |
| g) active ingredient² | **0.001-0.5%** |

### EXAMPLE 4: IN VIVO EFFICACY TESTING

The effectiveness of a cosmetic product can be proven through tests which can be performed
- *in vitro,* i.e., in the laboratory on cells or tissues, if the mechanism of action of the cosmetic is to be demonstrated; or
- *in vivo,* i.e., on people, to describe the effects visible over time.

*In vivo* tests can be self-assessment, sensory, clinical or instrumental.

The instrumental ones involve the use of machinery or probes to detect a certain parameter: by monitoring its variation over time, it is possible to have evidence of an improvement of the parameter after using the product.

The VISIA^{®} Skin Analysis System (https://www.canfieldsci.com/imaging-systems/visia-complexion-analysis) is the most innovative technology for the computerized analysis of the face, performed through some simple photographs acquired with three different types of light (cross-polarized, UV and with Canfield's RBX^{®} technology) and allows to evaluate the imperfections present on the skin surface.

A dedicated software is able to analyse the images, transform them into numerical values and allow an objective monitoring of the progress of the cosmetic treatment over time.

To evaluate the benefits of a cosmetic protocol, it is necessary to repeat the same analysis at a distance of time.

Depending on the cosmetic product being studied, it is possible to analyse its influence on the following parameters:
1. Spots: The instrument analyses the signs of obvious colour alteration on the surface of the skin, normally visible to the naked eye
2. Wrinkles: The instrument can differentiate between light and deep wrinkles, associated with a decrease in skin elasticity.
3. Skin texture: The instrument shows the uniformity of skin smoothness, highlighting 'lowered' areas and 'raised' areas which indicate variations on the skin surface.
4. Pores: the instrument detects the superficial circular openings of the ducts of the sweat glands
5. UV spots: The instrument highlights irregular areas below the skin surface that may be a consequence of sun damage.
6. Porphyrin deposits: these are bacterial excretions which can be deposited in the pores.
7. Brown spots: Signs of pigmentation and discolouration above and below the skin surface
8. Red areas: The instrument detects the presence of blood vessels below the skin surface. These areas are often associated with inflammation

It is therefore possible to accurately assess the condition of any person's skin: the data obtained will actually quantify the number of alterations present on the skin. The data obtained will also be compared with a database containing data related to a homogeneous population by age, phototype and sex with the subject analysed.

The efficacy of a mixture of active ingredients object of claim 1 of the present patent application has been validated by an *in vivo* test reported below.

### a) Study objective

Instrumental evaluation of the efficacy of the composition according to the invention.

### b) Product

Formulation for topical use (FACIAL SERUM of example 1) in which the sum of components a) + b) + c) + d) + e) is equal to 0.15% by weight on the total weight of said formulation and component f) is equal to 0.1% by weight on the total weight of said formulation.

### c) Sample of consumers

12 volunteers between 35 and 50 years of age with non-deep wrinkles, no active skin diseases, no allergies and/or intolerances to cosmetics or drugs were selected.

### d) Study design

The volunteers were asked to use the treatment at home twice a day for four consecutive weeks.

The timing at which the instrumental surveys were carried out with VISIA^{®} Complexion Analysis were as follows:
- Time 0 (before treatment start) on cleansed skin and without the application of any cosmetics.
- Time 4 (after 4 weeks of treatment). Both the texture and the condition of the wrinkles were evaluated.

### e) Instrumental evaluation

Through Visia^{®} Complexion Analysis, both the acquisition of images and an analysis of the parameters of interest were possible.

The measured data related to textures and wrinkles, the definition of which is given below, were subjected to statistical treatment to objectify any significant variation.
- *Texture:* analyses the smoothness of the skin, identifying colour gradations of the skin tone. In the image analysis related to texture, yellow represents the raised areas and blue the lowered ones.
- *Wrinkles:* VISIA^{®} identifies wrinkles based on their depth and their long, narrow shape. The dark green lines represent the most pronounced wrinkles, and the light green lines the thinnest wrinkles.

The numerical output taken into consideration was as follows: Absolute Score which relates the size/intensity of the elements found with respect to the area taken into account. A reduction in the Absolute Score at the end of treatment is indicative of an improvement in the parameters analysed.

### f) Results

### f1)T0 vs T4 weeks - skin texture.

Table 1 shows, for each of the volunteers, the Absolute Scores recorded at T0 (pre-treatment) and T4 (after 4 weeks of treatment). The Wilcoxon test for paired data was performed on the data obtained.

Being non-parametric data, such a test allows to establish if there is a statistically significant efficacy of the treatment (P=95%).

**Table 1**

| **Absolute Score - Texture** | | |
|---|---|---|
| | T0 | T4 |
| **Volunteer 1** | 9371 | 8693 |
| **Volunteer 2** | 8421 | 7139 |
| **Volunteer 3** | 8157 | 10142 |
| **Volunteer 4** | 3881 | 4950 |
| **Volunteer 5** | 11074 | 7849 |
| **Volunteer 6** | 15894 | 13782 |
| **Volunteer 7** | 6279 | 3605 |
| **Volunteer 8** | 7466 | 7711 |
| **Volunteer 9** | 9677 | 6474 |
| **Volunteer 10** | 13363 | 13174 |
| **Volunteer 11** | 20263 | 16579 |
| **Volunteer 12** | 7829 | 9150 |

In the whole panel of volunteers, the Wilcoxon test for paired data (P=95%) showed a statistically significant improvement in texture after 4 weeks of treatment.

The table shows that, in general, in 8 out of 12 cases there was a reduction in the Absolute Score - Texture value after 4 weeks of treatment with respect to the baseline condition.

### f2) Results T0 vs T4 Weeks - Skin Wrinkles

Table 2 shows, for each of the volunteers, the Absolute Scores recorded at T0 (pre-treatment) and T4 (after 4 weeks of treatment). The Wilcoxon test for paired data was performed on the data obtained.

Being non-parametric data, such a test allows to establish if there is a statistically significant efficacy of the treatment (P=95%).

**Table 2**

| **Absolute Score - Skin wrinkles** | | |
|---|---|---|
| | T0 | T4 |
| **Volunteer 1** | 27942 | 23645 |
| **Volunteer 2** | 17807 | 10028 |
| **Volunteer 3** | 22417 | 22526 |
| **Volunteer 4** | 3818 | 3013 |
| **Volunteer 5** | 8833 | 9099 |
| **Volunteer 6** | 43312 | 32468 |
| **Volunteer 7** | 7763 | 9355 |
| **Volunteer 8** | 9912 | 7132 |
| **Volunteer 9** | 22943 | 13927 |
| **Volunteer 10** | 14305 | 8003 |
| **Volunteer 11** | 43278 | 3783 |
| **Volunteer 12** | 21565 | 7621 |

In the whole panel of volunteers, the Wilcoxon test for paired data (P=95%) showed a statistically significant improvement in skin wrinkles after 4 weeks of treatment.

The table shows that, in general, in 9 out of 12 cases there was a reduction in the Absolute Score - Wrinkles value after 4 weeks of treatment with respect to the baseline condition.

### g) Conclusions

In the group of volunteers involved, the study showed a statistically significant improvement in the condition of wrinkles and skin texture after 4 weeks of treatment.

## Claims

1. Cosmetic compositions comprising
a) Sodium ascorbyl phosphate,
b) a pomegranate flower extract
c) a powdered extract of Quillaja Saponaria bark
d) an extract from the fruit of Kigelia africana
e) at least an amide of a tripeptide of formula Lys-Val-Lys with one or more C12-C18 straight-chain fatty acids.

2. Cosmetic compositions according to claim 1, wherein the sum of the quantities of
a) Sodium ascorbyl phosphate,
b) a pomegranate flower extract
c) a powdered extract of Quillaja Saponaria bark
d) an extract from the fruit of Kigelia africana
e) at least an amide of a tripeptide of formula Lys-Val-Lys with one or more C12-C18 straight-chain fatty acids
is between 0.02 and 0.5% by weight on the total weight of said cosmetic compositions.

3. Cosmetic compositions according to claim 1 or 2, wherein the sum of the quantities of
a) Sodium ascorbyl phosphate,
b) a pomegranate flower extract
c) a powdered extract of Quillaja Saponaria bark
d) an extract from the fruit of Kigelia africana
e) at least an amide of a tripeptide of formula Lys-Val-Lys with one or more C12-C18 straight-chain fatty acids
is between 0.06 and 0.08%, preferably equal to 0.0701% by weight on the total weight of said compositions, when the cosmetic compositions are in the form of a cream.

4. Cosmetic compositions according to claim 1 or 2, wherein the sum of the quantities of
a) Sodium ascorbyl phosphate,
b) a pomegranate flower extract
c) a powdered extract of Quillaja Saponaria bark
d) an extract from the fruit of Kigelia africana
e) at least an amide of a tripeptide of formula Lys-Val-Lys with one or more C12-C18 straight-chain fatty acids
is between 0.09% and 0.2%, preferably equal to 0.15% by weight on the total weight of said compositions, when the cosmetic compositions are in the form of serum.

5. Compositions according to any one of claims from 1 to 4, wherein the at least an amide of a tripeptide Lys-Val-Lys with one or more carboxylic acids is an amide of the tripeptide of formula Lys-Val-Lys with a fatty acid, said fatty acid being the palmitic acid.

6. Composition according to claim 5, wherein the amide of the tripeptide of formula Lys-Val-Lys with palmitic acid is N-Palmitoyl-L-lysyl-L-valyl-L-lysine trifluoroacetate (1:2) of formula (I):

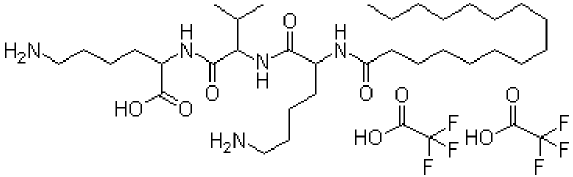

7. Cosmetic compositions according to any one of claims from 1 to 6, further comprising
f) hyaluronic acid or a biologically acceptable salt thereof, said biologically acceptable salt being preferably a metal alkali salt, preferably sodium salt.

8. Cosmetic compositions according to claim 7, wherein the hyaluronic acid consist of:
- hyaluronic acid with a weight average molecular weight < 10,000 Da, or
- hyaluronic acid with a weight average molecular weight between 10,000 and 1,000,000 Da, or
- hyaluronic acid with a weight average molecular weight > 1,000,000 Da, or
- combinations of two or three of the above mentioned hyaluronic acid types.

9. Cosmetic compositions according to claim 8, wherein the total concentrations of hyaluronic acid are preferably comprised between 0.01% and 1% by weight on the total weight of said cosmetic compositions.

10. Cosmetic compositions according to any one of claims from 1 to 4 in the form of a cream, comprising
a) Sodium ascorbyl phosphate,
b) a pomegranate flower extract
c) a powdered extract of Quillaja Saponaria bark
d) an extract from the fruit of Kigelia africana
e) at least an amide of a tripeptide of formula Lys-Val-Lys with one or more C12-C18 straight-chain fatty acids
f) hyaluronic acid or a biologically acceptable salt thereof, said biologically acceptable salt being preferably a metal alkali salt, preferably sodium salt, and
g) an active ingredient selected from the group consisting of: Ascorbyl glucoside, N-acetyl-L-lysyl-L-alpha-aspartyl-L-valyl-L-tyrosine, *Akebia Quinata* extract, Niacinamide and *Salvia miltiorrhiza* leaf extract.

11. Non therapeutic use of the cosmetic compositions according to any one of claims from 1 to 10, for the prevention of skin ageing.

12. Non therapeutic use of the composition according to claim 10, to firm the skin, further containing as active ingredient g) ascorbil-glucoside.

13. Non therapeutic use of the composition according to claim 10 to firm face and neck, further containing as active ingredient g) N-acetyl-L-lysyl-L-alpha-aspartyl-L-valyl-L-tyrosine.

14. Non therapeutic use of the composition according to claim 10 to firm and nourish the skin, further containing as active ingredient g) a combination of niacinamide and *Salvia miltiorrhiza* leaf extract.

15. Non therapeutic use of the composition according to claim 10 as a lifting agent to firm the skin, further containing as active ingredient g) *Akebia quinata*

## Patentansprüche

1. Kosmetische Zusammensetzungen, umfassend
a) Natriumascorbylphosphat,
b) einem Granatapfelblütenextrakt
c) einem pulverisierten Extrakt aus Quillaja-Saponaria-Rinde
d) einem Extrakt aus der Frucht von Kigelia africana
e) mindestens ein Amid eines Tripeptids von Formel Lys-Val-Lys mit einer oder mehreren geradkettigen C12-C18-Fettsäuren.

2. Kosmetische Zusammensetzungen nach Anspruch 1, wobei die Summe der Mengen an
a) Natriumascorbylphosphat,
b) einem Granatapfelblütenextrakt
c) einem pulverisierten Extrakt aus Quillaja-Saponaria-Rinde
d) einem Extrakt aus der Frucht von Kigelia africana
e) mindestens einem Amid eines Tripeptids der Formel Lys-Val-Lys mit einer oder mehreren geradkettigen C12-C18-Fettsäuren
zwischen 0,02 und 0,5 Gewichts-%, bezogen auf das Gesamtgewicht der kosmetischen Zusammensetzungen, ist.

3. Kosmetische Zusammensetzungen nach Anspruch 1 oder 2, wobei die Summe der Mengen an
a) Natriumascorbylphosphat,
b) einem Granatapfelblütenextrakt
c) einem pulverisierten Extrakt aus Quillaja-Saponaria-Rinde
d) einem Extrakt aus der Frucht von Kigelia africana
e) mindestens einem Amid eines Tripeptids der Formel Lys-Val-Lys mit einer oder mehreren geradkettigen C12-C18-Fettsäuren
zwischen 0,06 und 0,08 Gewichts-%, vorzugsweise gleich wie 0,0701 Gewichts-%, bezogen auf das Gesamtgewicht der Zusammensetzungen ist, wenn die kosmetischen Zusammensetzungen in Form einer Creme sind.

4. Kosmetische Zusammensetzungen nach Anspruch 1 oder 2, wobei die Summe der Mengen an
a) Natriumascorbylphosphat,
b) einem Granatapfelblütenextrakt
c) einem pulverisierten Extrakt aus Quillaja-Saponaria-Rinde
d) einem Extrakt aus der Frucht von Kigelia africana
e) mindestens einem Amid eines Tripeptids der Formel Lys-Val-Lys mit einer oder mehreren geradkettigen C12-C18-Fettsäuren
zwischen 0,09 und 0,2 %, vorzugsweise gleich 0,15 Gewichts-%, bezogen auf das Gesamtgewicht der Zusammensetzungen ist, wenn die kosmetischen Zusammensetzungen in Form von Serum sind.

5. Zusammensetzungen nach einem der Ansprüche 1 bis 4, wobei das mindestens eine Amid eines Tripeptids Lys-Val-Lys mit einer oder mehreren Carbonsäuren ein Amid des Tripeptids von Formel Lys-Val-Lys mit einer Fettsäure ist, wobei die Fettsäure die Palmitinsäure ist.

6. Zusammensetzung nach Anspruch 5, wobei das Amid des Tripeptids von Formel Lys-Val-Lys mit Palmitinsäure N-Palmitoyl-L-lysyl-L-valyl-L-lysin-trifluoracetat (1:2) von Formel (I) ist:

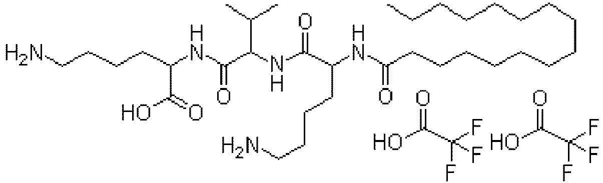

7. Kosmetische Zusammensetzungen nach einem der Ansprüche 1 bis 6, ferner umfassend:
f) Hyaluronsäure oder ein biologisch annehmbares Salz davon, wobei das biologisch annehmbare Salz vorzugsweise ein Metallalkalisalz, vorzugsweise Natriumsalz, ist.

8. Kosmetische Zusammensetzungen nach Anspruch 7, wobei die Hyaluronsäure aus Folgendem besteht:
- Hyaluronsäure mit einem gewichtsmittleren Molekulargewicht <10.000 Da, oder
- Hyaluronsäure mit einem gewichtsmittleren Molekulargewicht zwischen 10.000 und 1.000.000 Da, oder
- Hyaluronsäure mit einem gewichtsmittleren Molekulargewicht >1.000.000 Da, oder
- Kombinationen von zwei oder drei der oben genannten Hyaluronsäure-Arten.

9. Kosmetische Zusammensetzungen nach Anspruch 8, wobei die Gesamtkonzentrationen an Hyaluronsäure vorzugsweise zwischen 0,01 und 1 Gewichts-%, bezogen auf das Gesamtgewicht der kosmetischen Zusammensetzungen, sind.

10. Kosmetische Zusammensetzungen nach einem der Ansprüche 1 bis 4 in Form einer Creme, umfassend
a) Natriumascorbylphosphat,
b) einem Granatapfelblütenextrakt
c) einem pulverisierten Extrakt aus Quillaja-Saponaria-Rinde
d) einem Extrakt aus der Frucht von Kigelia africana
e) mindestens einem Amid eines Tripeptids der Formel Lys-Val-Lys mit einer oder mehreren geradkettigen C12-C18-Fettsäuren
f) Hyaluronsäure oder ein biologisch annehmbares Salz davon, wobei das biologisch annehmbare Salz vorzugsweise ein Metallalkalisalz, vorzugsweise Natriumsalz, ist, und
g) einen Wirkstoff, ausgewählt aus der Gruppe, bestehend aus: Ascorbylglucosid, N-Acetyl-L-lysyl-L-alpha-aspartyl-L-valyl-L-tyrosin, Extrakt von *Akebia Quinata,* Niacinamid und Blätterextrakt von *Salvia miltiorrhiza.*

11. Nicht-therapeutische Verwendung der kosmetischen Zusammensetzungen nach einem der Ansprüche 1 bis 10 zum Verhindern von Hautalterung.

12. Nicht-therapeutische Verwendung der Zusammensetzung nach Anspruch 10, um die Haut zu straffen, ferner enthaltend Ascorbil-Glucosid als Wirkstoff g).

13. Nicht-therapeutische Verwendung der Zusammensetzung nach Anspruch 10, um Gesicht und Hals zu straffen, ferner enthaltend g) N-Acetyl-L-lysyl-L-alpha-aspartyl-L-valyl-L-tyrosin als Wirkstoff.

14. Nicht-therapeutische Verwendung der Zusammensetzung nach Anspruch 10, um die Haut zu straffen und zu pflegen, ferner enthaltend g) eine Kombination aus Niacinamid und Blätterextrakt von *Salvia miltiorrhiza* als Wirkstoff.

15. Nicht-therapeutische Verwendung der Zusammensetzung nach Anspruch 10 als Lifting-Mittel, um die Haut zu straffen, ferner enthaltend g) *Akebia quinata* als Wirkstoff.

## Revendications

1. Compositions cosmétiques comprenant
a) du phosphate d'ascorbyle de sodium,
b) un extrait de fleur de grenade
c) un extrait en poudre d'écorce de Quillaja Saponaria
d) un extrait du fruit de Kigelia africana
e) au moins un amide d'un tripeptide de formule Lys-Val-Lys avec un ou plusieurs acides gras à chaîne droite en C12 à C18.

2. Compositions cosmétiques selon la revendication 1, dans lesquelles la somme des quantités de
a) phosphate d'ascorbyle de sodium,
b) un extrait de fleur de grenade
c) un extrait en poudre d'écorce de Quillaja Saponaria
d) un extrait du fruit de Kigelia africana
e) au moins un amide d'un tripeptide de formule Lys-Val-Lys avec un ou plusieurs acides gras à chaîne droite en C12 à C18
est comprise entre 0,02 et 0,5 % en poids par rapport au poids total desdites compositions cosmétiques.

3. Compositions cosmétiques selon la revendication 1 ou 2, dans lesquelles la somme des quantités de
a) phosphate d'ascorbyle de sodium,
b) un extrait de fleur de grenade
c) un extrait en poudre d'écorce de Quillaja Saponaria
d) un extrait du fruit de Kigelia africana
e) au moins un amide d'un tripeptide de formule Lys-Val-Lys avec un ou plusieurs acides gras à chaîne droite en C12 à C18
est comprise entre 0,06 et 0,08 %, de préférence égale à 0,0701 % en poids par rapport au poids total desdites compositions, lorsque les compositions cosmétiques se présentent sous forme de crème.

4. Compositions cosmétiques selon la revendication 1 ou 2, dans lesquelles la somme des quantités de
a) phosphate d'ascorbyle de sodium,
b) un extrait de fleur de grenade
c) un extrait en poudre d'écorce de Quillaja Saponaria
d) un extrait du fruit de Kigelia africana
e) au moins un amide d'un tripeptide de formule Lys-Val-Lys avec un ou plusieurs acides gras à chaîne droite en C12 à C18
est comprise entre 0,09 % et 0,2 %, de préférence égale à 0,15 % en poids par rapport au poids total desdites compositions, lorsque les compositions cosmétiques sont sous forme de sérum.

5. Compositions selon l'une quelconque des revendications 1 à 4, dans lesquelles l'au moins un amide d'un tripeptide Lys-Val-Lys avec un ou plusieurs acides carboxyliques est un amide du tripeptide de formule Lys-Val-Lys avec un acide gras, ledit acide gras étant l'acide palmitique.

6. Compositions selon la revendication 5, dans lesquelles l'amide du tripeptide de formule Lys-Val-Lys avec l'acide palmitique est le trifluoroacétate de N-Palmitoyl-L-lysyl-L-valyl-L-lysine (1:2) de formule (I) :

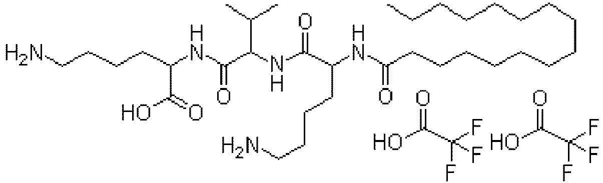

7. Compositions cosmétiques selon l'une quelconque des revendications 1 à 6, comprenant en outre :
f) de l'acide hyaluronique ou un sel biologiquement acceptable de celui-ci, ledit sel biologiquement acceptable étant de préférence un sel alcalin métallique, de préférence un sel de sodium.

8. Compositions cosmétiques selon la revendication 7, dans lesquelles l'acide hyaluronique est constitué de :
- acide hyaluronique avec un poids moléculaire moyen en poids < 10 000 Da, ou
- acide hyaluronique avec un poids moléculaire moyen en poids compris entre 10 000 et 1 000 000 Da, ou
- acide hyaluronique avec un poids moléculaire moyen en poids > 1 000 000 Da, ou
- combinaisons de deux ou trois des types d'acide hyaluronique mentionnés ci-dessus.

9. Compositions cosmétiques selon la revendication 8, dans lesquelles les concentrations totales en acide hyaluronique sont de préférence comprises entre 0,01 % et 1 % en poids par rapport au poids total des compositions cosmétiques.

10. Compositions cosmétiques selon l'une quelconque des revendications 1 à 4 sous forme de crème, comprenant
a) du phosphate d'ascorbyle de sodium,
b) un extrait de fleur de grenade
c) un extrait en poudre d'écorce de Quillaja Saponaria
d) un extrait du fruit de Kigelia africana
e) au moins un amide d'un tripeptide de formule Lys-Val-Lys avec un ou plusieurs acides gras à chaîne droite en C12 à C18
f) de l'acide hyaluronique ou un sel biologiquement acceptable de celui-ci, ledit sel biologiquement acceptable étant de préférence un sel alcalin métallique, de préférence un sel de sodium, et
g) un principe actif sélectionné dans le groupe constitué par : glucoside d'ascorbyle, N-acétyl-L-lysyl-L-alpha-aspartyl-L-valyl-L-tyrosine, extrait d*'Akebia Quinata,* niacinamide et extrait de feuille de *Salvia miltiorrhiza.*

11. Utilisation non thérapeutique des compositions cosmétiques selon l'une quelconque des revendications 1 à 10, pour la prévention du vieillissement cutané.

12. Utilisation non thérapeutique de la composition selon la revendication 10, pour raffermir la peau, contenant en outre comme principe actif g) du glucoside d'ascorbyle.

13. Utilisation non thérapeutique de la composition selon la revendication 10 pour raffermir le visage et le cou, contenant en outre comme principe actif g) la N-acétyl-L-lysyl-L-alpha-aspartyl-L-valyl-L-tyrosine.

14. Utilisation non thérapeutique de la composition selon la revendication 10 pour raffermir et nourrir la peau, contenant en outre comme principe actif g) une combinaison de niacinamide et d'extrait de feuille de *Salvia miltiorrhiza.*

15. Utilisation non thérapeutique de la composition selon la revendication 10 en tant qu'agent de lissage pour raffermir la peau, contenant en outre en tant que principe actif g) de l*'Akebia quinata.*
